(19) Europäisches Patentamt
European Patent Office
Office européen des brevets

(11) EP 1 018 346 B1

(12) EUROPEAN PATENT SPECIFICATION

(45) Date of publication and mention of the grant of the patent:
**01.09.2004 Bulletin 2004/36**

(51) Int Cl.[7]: **A61M 1/36**, B01D 67/00, B01D 39/16

(21) Application number: **99310618.6**

(22) Date of filing: **29.12.1999**

# (54) Leucocyte filter and method for manufacturing same

Leukozytenfilter und Verfahren zu seiner Herstellung

Filtre de leucocytes et son procédé de fabrication

(84) Designated Contracting States:
**AT BE CH CY DE DK ES FI FR GB GR IE IT LI LU MC NL PT SE**

(30) Priority: **07.01.1999 JP 175699**

(43) Date of publication of application:
**12.07.2000 Bulletin 2000/28**

(73) Proprietor: **TERUMO KABUSHIKI KAISHA**
**Tokyo 151-0072 (JP)**

(72) Inventor: **Motomura, Tadahiro**
**Ashigarakami-gun, Kanagawa (JP)**

(74) Representative: **Harrison, David Christopher et al**
**Mewburn Ellis LLP**
**York House**
**23 Kingsway**
**London WC2B 6HP (GB)**

(56) References cited:
**EP-A- 0 856 319**
**US-A- 2 723 256**
**US-A- 3 864 317**
**US-A- 4 936 998**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

This invention relates to a leukocyte eliminating filter and a method for manufacturing the same and, more particularly, to a cationized and hydrophilized leukocyte-eliminating filter which is made from a porous film, and to a manufacturing method of such a filter, wherein the surface of the porous film is cationized. According to the invention, the surface has (through coating, for instance) a hydrophilic polymer comprising an alkylsulfate or alkylsulfonate of primary, secondary, tertiary or quaternary amine, thereby modifying the surface of the porous film so as to cationize and hydrophilize the filter.

Blood transfusion involves the elimination of inactivate leukocyte through a centrifugal separation, a radiation exposure or filtration in order to prevent various side reactions, such as the induction of GVHD (Graft Versus Host Disease), brought about mainly due to the presence of leukocyte, or to prevent infectious disease that may be caused by a virus-infected leukocyte. Elimination of leukocyte by means of filtration is widely adopted as it can be executed at a patient's bedside and is simple and low-cost.

It is desired when eliminating leukocyte at a patient's bedside to employ a filter which is not only an efficient remover of leukocytes but is also capable of performing smooth filtration. Such a filter can be obtained by modifying the surface of the porous film of the filter. This surface modification involves not only rendering the surface to be easily adherable to leukocyte and adjusting the pore size of the porous film for the physical elimination of leukocyte, but also to render the surface hydrophilic.

It is known from US-A-3242073 and 3352424 to increase adhesion to the filter by cationizing the surface of the filter, since the surface of a cell such as a leukocyte is electronegatively charged. The use of polymer coated leukocyte filters is known from US 4 936 998.

Further, when the surface of the leukocyte eliminating filter is rendered to be hydrophilic, blood or a blood preparation is allowed to pass through the filter with little resistance, thereby making it possible to realize a smooth filtration thereof. An index of the hydrophilicity is the critical wet surface tension thereof (hereinafter referred to as CWST, which will be explained below in detail), is known.

Therefore, an object of the present invention is to provide a leukocyte filter which can be easily manufactured and a method for manufacturing it, which can be realized through the cationization and hydrophilization of the surface of a substrate constituting the filter.

It has been found as a result of intensive studies by the present inventors that the aforementioned objects can be realized by coating a substrate of a leukocyte removal filter with a polymer having a chemical structure represented by of the following formulae (I) or (II);

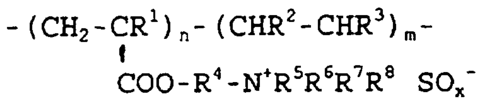

(I)

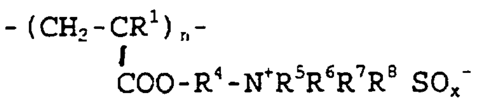

(II)

wherein $R^1$, $R^3$, $R^4$, $R^5$, $R^6$ and $R^7$ may be the same or different and are individually hydrogen atom or an alkyl group having 1 to 4 carbon atoms; $R^2$ is $CONH_2$ or COOH; $R^8$ is an alkyl group having 1 to 12 carbon atoms; and x is 3 or 4. These polymers are known as antistatic agents (see e.g. US 2 723 256).

This invention will be further explained as follows.

The porous substrate for the leukocyte filter may be a porous body such as a spongy or fibrous body, a film, a hollow fiber, an unwoven fabric, a woven fabric, or a composite thereof. The porosity of the substrate should preferably be in the range of 75% to 95%, more preferably in the range of 80% to 95%, and the pore diameter thereof as measured by means of the mercury injecting method should preferably be in the range of 0.1 to 30µm, more preferably in the range of 2 to 20µm. The reasons for these features of the substrate are as follows. If the porosity of the substrate is 75% or more, it becomes possible to minimize filtration time, while if the porosity of the substrate is 95% or less, it becomes possible to ensure strength of the filter. On the other hand, if the pore diameter of the substrate is less than 0.1µm, the clogging of the filter be easily caused during operation, thus making the filter useless, while if the pore diameter of the substrate is more than 30µm, the frequency of contact between the blood or the leukocyte in blood

preparation and the filter deteriorates, thus reducing the ratio of leukocyte trapped.

The filter should preferably be constructed such that the substrate itself is cationic or the surface thereof is cationized. Because the cell surface of leukocyte is electronegatively charged, it is easier to trap the leukocyte if the surface of the leukocyte filter is made cationic.

For the porous substrate of the leukocyte eliminating filter, it is possible to employ a fibrous body or a spongy body made from a natural polymer such as cotton and hemp; a synthetic polymer such as polyester (e.g. PET), polyacrylonitrile, polyolefin, polyolefin halide, polyurethane, polysulfone, polyether sulfone, poly(metha)acrylate, ethylene-polyvinyl alcohol copolymer, or butadiene-acrylonitrile copolymer; or a mixture thereof. In view of workability and compatibility with blood however, a spongy polyurethane porous body is most preferable.

However, since these substrates for the blood filter, which are represented by polyurethane for instance, are generally highly hydrophobic, though they can give an excellent leukocyte elimination due to their excellent workability and blood compatibility as mentioned above, it is rather difficult for blood to smoothly pass through the pores of the fibrous or spongy body thereof. We conclude that some sort of hydrophilization treatment of these substrates needs to be performed.

If the hydrophilization treatment can be conducted simultaneous with cationization treatment, these treatments can be accomplished once for all, thus making it possible to simplify working and to improve production efficiency. According to this invention, it is now possible, through a simple operation, to improve the hydrophilicity of the substrate and at the same time to promote the leukocyte eliminating performance of the substrate, thus increasing the production capacity of the filter.

Following are specific examples of coating agent suitable to be employed in this invention. These are high molecular compounds which can be represented by the following general formulae (I) or (II)given and defined above.

Polymers represented by the aforementioned general formulae (I) and (II) where an alkylsulfate ion or an alkylsulfonate ion is employed as a counter ion can be synthesized by employing the methods set forth in US-A-2,723,256 for instance. They are also commercially available since they are marketed as antistatic agents. It is also possible to employ any other known techniques for the manufacture of these polymers represented by the aforementioned general formulas (I) and (II).

The ratio between n and m in the general formula (I) should preferably be in the range of 99:1 to 90:10 (n:m).

The molecular weight of the polymers represented by the aforementioned general formulas (I) and (II) should preferably be in the range of 650,000 to 1,050,000, more preferably in the range of 750,000 to 950,000. If the molecular weight is limited within this range, elution of the polymer can be minimized.

The method of retaining these compounds on the substrate of the filter, i.e. the method for manufacturing the leukocyte eliminating filter of this invention, it can be performed by a simple process wherein one or more of the aforementioned coating agents is coated on the porous film by means of coating, dipping, spray or spin-coating, and then, the coated layer is heat-dried at, e.g., a temperature of 60° to 100°C. It may be more preferable to perform the washing of the coated layer after the aforementioned heat-drying step so as to wash out any eluted material.

Alternatively, it is possible to employ a process wherein a quaternary amine ester of polyacrylic acid or of a copolymer of acrylic acid with acrylamide is coated on the substrate of the leukocyte eliminating filter, and then, the coated layer is heat-dried, the resultant dried layer being subsequently treated with an aqueous solution of sodium alkylsulfonate, or with an aqueous solution of sodium alkylsulfate.

CWST, i.e. an index representing the hyrophilicity of the leukocyte-eliminating filters is a value which can be determined by the method which is set forth in EP-A-397403, and is as follows.

First, a plurality of aqueous solutions of calcium hydroxide, sodium hydroxide, calcium chloride, sodium nitrate, acetic acid, ethanol, etc. of various concentrations such that the surface tensions thereof are varied by 2 to 4 dyn/cm are prepared. Then, these aqueous solutions of various surface tensions are successively allowed to drop in order of surface tension (starting from the lowest)in does of 10 drops on the surface of a leukocyte eliminating filter. These drops of aqueous solutions are then left to stand for 10 minutes. When not less than 9 out of 10 drops are absorbed by the leukocyte eliminating filter after the aforementioned 10-minute wait, the solution is determined as being a wet state, while when less than nine out of 10 drops are absorbed by the leukocyte eliminating filter after the aforementioned 10-minute wait, the solution is determined as being a non-wet state. The measurement is carried on in order of surface tension starting from that of the lowest surface tension, the wet state and the non-wet state can be observed. An average of the values of the surface tension of the aqueous solutions where the wet state is observed and of the surface tension of the aqueous solution where the non-wet state is observed is defined as being the CWST value of the surface of the filter. For example, if the wet state is observed for a solution having a surface tension of 64 dyn/cm, and the non-wet state is observed for a solution having a surface tension of 66 dyn/cm, the value of CWST of the surface is 65 dyn/cm.

The value of CWST of the surface of the leukocyte eliminating filter according to this invention should preferably be in the range of 90 to 95 dyn/cm. If the value of CWST exceeds 95 dyn/cm, there is no substantial improvement in the penetration of blood into the filter. On the other hand, if the value of this CWST is 90 dyn/cm or more, high

penetration speed of blood could be expected, so that a priming operation would not be required to be performed in advance and the time required for the filtration can be shortened.

The invention will now be explained with reference to the following Examples, which do not restrict the scope of the invention.

The measurement of the CWST in the following Examples and Comparative Examples was conducted in the same manner as explained above.

The measurements of leukocyte eliminating ratio and platelet recovery ratio were performed as follows. A 25 mm diameter piece of the porous film which had been cationized and hydrophilized was attached to a module having an inlet port and an outlet port to simulate a leukocyte eliminating filter. Fresh blood collected from a healthy volunteer, with CPD added, was adjusted to prepare a concentrated solution of erythrocyte. Then, 5mL of this concentrated solution of erythrocyte was allowed to flow down to the leukocyte eliminating filter from 10cm above it, and the quantities of leukocyte and blood plasma before and after the passage of the concentrated solution of erythrocyte through the filter were measured. Based on the measured results, the elimination ratios of leukocyte and platelet were determined from the following formulae.

Leukocyte elimination ratio = (1-(number of

leukocyte after filtration/number of leukocyte before

filtration)) x 100.

Platelet elimination ratio = (1-(number of

platelet after filtration/number of platelet before

filtration)) x 100.

The leukocyte and platelet counts were determined by an automatic blood corpuscle measuring apparatus (SYSMEX E-9000(TM) from Toua Iryou Denshi Co., Ltd.).

Example 1

As a substrate for the leukocyte eliminating filter, a spongy polyurethane porous film (Rubycell; Toyo Polymer Co., Ltd.) 5.2 microns (µm) in average pore diameter, 86% in porosity and 1.2mm in thickness was employed.

This porous film was subjected to ultrasonic cleaning in hexane for one hour, and after being dried, dipped in an aqueous solution (0.3w/w%) of a compound polyacrylamide-poly(acryloyloxy) ethyldiethylmethyl ammonium methylsulfonate (Calgon K400, tradename; Calgon Co., Ltd.)see formula 1:

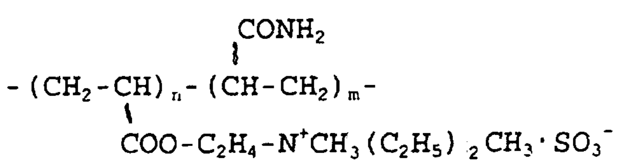

(1)

Subsequently, the porous film was taken out of the aqueous solution and heat-treated for two hours at a temperature of 80°C, after which the porous film was shower-washed for four hours at room temperature and then, allowed to dry.

Thereafter, the leukocyte elimination ratio and the platelet elimination ratio were respectively determined as above and by making use of the porous film prepared as explained above. The time interval between the initiation of blood flow and the initiation of oozing of the blood from the bottom surface (filtrate side) of the porous film after the porous film was completely filled with the blood (or wetting time) was also measured as a criterion of the hydrophilicity of the porous film. Then, the CWST of the porous film prepared as explained above was also measured according to the aforementioned method.

The results obtained are shown in Table 1 and Table 2.

Additionally, elution tests of the porous film prepared as explained above were also performed to confirm the safety of the porous film. The elution tests of the porous film on the following five items were performed according to the specification standard for medical appliances and the standard for disposable transfusion sets and blood sets (Notice No.301 from the Ministry of Health and Welfare of Japan, 1970) ((1): External appearance; (2): pH; (3): potassium permanganate-reducing substances; (4): residues on evaporation; (5): heavy metals). It was confirmed as a result of these analyses that the porous film was capable of meeting these standards on all of the aforementioned items.

Example 2

The porous film obtained in Example 1 was subjected to ultrasonic cleaning in hexane for one hour, and after being dried, dipped in an aqueous solution (0.3w/w%) of the compound polyacrylate-poly2-(acryloyloxy) ethyldiethylmethyl ammonium methylsulfate (Eletat U52(™); Ippousha Yushi Manufacturing Co., Ltd.)-see formula 2.

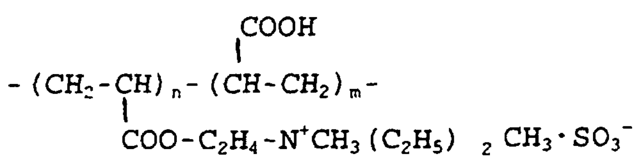

(2)

Subsequently, the porous film was taken out of the aqueous solution and heat-treated for two hours at a temperature of 80°C, after which it was shower-washed for four hours at room temperature and then allowed to dry.

Thereafter, the leukocyte elimination ratio and the platelet elimination ratio were respectively determined as above and by making use of the porous film prepared as explained above. The time interval between the initiation of blood flow and the initiation of oozing of the blood from the bottom surface (filtrate side) of the porous film after the porous film was completely filled with the blood (or wetting time) was also measured as a criterion of the hydrophilicity of the porous film. The CWST of the porous film prepared as explained above was also measured according to the aforementioned method.

The results obtained are shown in Table 1 and Table 2.

Additionally, when the above elution tests were performed the porous film was confirmed as being capable of meeting the required standards on all of the aforementioned items.

Example 3

The porous film obtained in Example 1 was subjected to ultrasonic cleaning in hexane for one hour, and after being dried, dipped in a 2-propanol solution (lw/w%) of a compound poly(acryloyloxy) ethyldiethylmethyl ammonium methylsulfonate (Eletat U52(™); Ippousha Yushi Industries Co., Ltd.) - see formula 3.

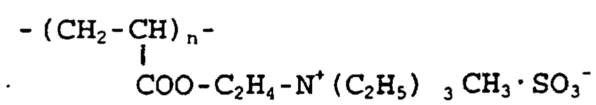

(3)

Subsequently, the porous film was taken out of the aqueous solution and heat-treated for two hours at a temperature of 80°C, after which it was shower-washed for four hours at room temperature and then, allowed to dry.

Thereafter, the leukocyte elimination ratio and the platelet elimination ratio were respectively determined as above and by making use of the porous film prepared as explained above. In this case, the time interval between the initiation of blood flow and the initiation of oozing of the blood from the bottom surface (filtrate side) of the porous film after the porous film was completely filled with the blood (or wetting time) was also measured as a criterion of the hydrophilicity of the porous film. The CWST of the porous film prepared as explained above was also measured according to the aforementioned method.

The results obtained are shown in Table 1 and Table 2.

Additionally, when the above elution tests were performed the porous film was confirmed as being capable of meeting the required standards on all of the aforementioned items.

Comparative Example 1

The same kind of polyurethane porous film as employed in Example 1 was subjected to ultrasonic cleaning in hexane for one hour, and then allowed to dry.

Subsequently, by making use of this untreated porous film as a substrate for a leukocyte eliminating film, the leukocyte elimination ratio and the platelet elimination ratio were respectively determined according to the same procedures as explained in Example 1. The time interval between the initiation of blood flow and the initiation of oozing of the blood from the bottom surface (filtrate side) of the porous film after the porous film was completely filled with the blood (or wetting time) was also measured together with the CWST of the porous film.

The results obtained are shown in Table 1 and Table 2.

Comparative Example 2

Polyacrylamide-poly2-(acryloyloxy) ethyldiethylmethyl ammonium methylsulfate (Calgon K 400(™); Calgon Co., Ltd.) was refined by allowing the polymer to repeatedly reprecipitate three times in tetrahydrofuran. This refined polymer was formulated into a 3 wt% aqueous solution thereof. Then, 50mL of this aqueous solution was allowed to pass through Amberlite IRA 402BL Cl (Organo Co., Ltd.) which had been charged into a 50mm x 300mm chromatographic column so as to allow the counter ion thereof to be exchanged with Cl-. Thereafter, the resultant compound was refined by repeatedly allowing it to reprecipitate three times in tetrahydrofuran, thereby synthesizing Calgon K 400 represented by the following formula (4) where the counter ion is constituted by Cl-ion.

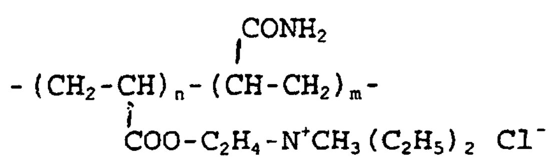

(4)

Then, the porous film of Example 1 was subjected to ultrasonic cleaning in hexane for one hour, and after being dried, dipped in an aqueous solution (0.3w/w%) of Calgon K 400 represented by the formula (4) where the counter ion is constituted by Cl- ion. Subsequently, it was taken out of the aqueous solution and heat-treated for two hours at a temperature of 80'C, after which it was shower-washed for four hours at room temperature and then allowed to dry.

Thereafter, the leukocyte elimination ratio and the platelet elimination ratio were respectively determined as aboveand by making use of the porous film prepared as explained above. The time interval between the initiation of blood flow and the initiation of oozing of the blood from the bottom surface (filtrate side) of the porous film after the porous film was completely filled with the blood (or wetting time) was also measured as a criterion for the hydrophilicity of the porous film.

The results obtained are shown in Table 1 and Table 2.

Example 4

The polymer obtained in Comparative Example 2 where the counter ion was constituted by Cl- ion was formulated into a 3 wt% aqueous solution thereof. Then, a 10wt% aqueous solution of chlorine lauryl sulfate was added to 50mL of this aqueous solution. As a result, since an insoluble polymer where the Cl ion was substituted by lauryl sulfate ion was gradually allowed to precipitate, the precipitated substance was recovered by means of filtration, thereby synthesizing Calgon K 400 represented by the following formula (5) where the counter ion is constituted by alkyl sulfate ion.

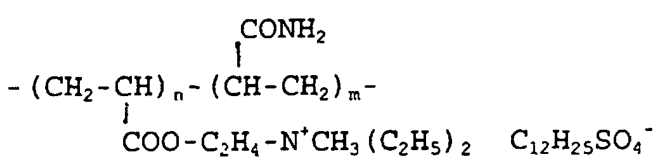

(5)

The porous film of Example 1 was subjected to ultrasonic cleaning in hexane for one hour, and after being

dried, was dipped in an aqueous solution (0.3w/w%) of Calgon K 400 represented by the following formula (5) where the counter ion was constituted by lauryl sulfate ion. Subsequently, the porous film was taken out of the aqueous solution and heat-treated for two hours at a temperature of 80°C, after which the porous film was shower-washed for four hours at room temperature and then allowed to dry.

Thereafter, the leukocyte elimination ratio and the platelet elimination ratio were respectively determined as above and by making use of the porous film prepared as explained above. The time interval between the initiation of blood flow and the initiation of oozing of the blood from the bottom surface (filtrate side) of the porous film after the porous film was completely filled with the blood (or wetting time) was also measured as a criterion for the hydrophilicity of the porous film.

The results obtained are shown in Table 1 and Table 2.

Example 5

A 30g/m$^2$ polyester unwoven fabric, which has 0.5mm thickness and 30 μm maximum pore diameter, made from fibers having a diameter of 1 to 5 μm, (Tohnen Tapils Co., Ltd.) was dipped into a 3 w/w% aqueous coating solution of polyacrylamide-poly2-(acryloyloxy) ethyldiethylmethyl ammonium methylsulfate (Calgon K 400(™); tradename; Calgon Co., Ltd.). Then, the unwoven fabric was heat-dried for two hours at a temperature of 80°C, after which the unwoven fabric was shower-washed for four hours at room temperature to remove eluted substances and then allowed to dry.

Thereafter, the leukocyte elimination ratio and the platelet elimination ratio were respectively determined as above and by making use of the unwoven fabric prepared as explained above.

The results obtained are shown in Table 1 and Table 2.

Comparative Example 3

A 30g/m$^2$ polyester unwoven fabric, which has 0.5mm thickness and 30 μm maximum pore diameter, made from fibers having a diameter of 1 to 5 μm, (Tohnen Tapils Co., Ltd.) was shower-washed for four hours at room temperature to remove the substances eluted from the unwoven fabric, and then allowed to dry.

Thereafter, the leukocyte eliminating ratio and the platelet eliminating ratio were respectively determined according to the aforementioned process and by making use of the unwoven fabric prepared as explained above.

The results obtained are shown in Table 1 and Table 2.

Table 1

| Example | CWST (dyn/cm) | Reflection absorbency | Leukocyte eliminated (%) | Platelet eliminated (%) |
|---|---|---|---|---|
| 1 | 90 | 0.66 | 99.7 | 99.1 |
| 2 | 90 | 0.97 | 99.5 | 99.1 |
| 3 | 91 | 0.98 | 99.7 | 99.2 |
| 4 | 90 | 0.376 | 99.9 | 99.3 |
| 5 | -- | 0.350 | 88.5 | 87.0 |
| Comparative Example | | | | |
| 1 | 72 | 0.03 | 87.0 | 21.2 |
| 2 | 72 | 0.124 | 87.8 | 21.8 |
| 3 | -- | 0.06 | 61.0 | 35.0 |

Table 2

| Example | Wet time (sec.) |
|---|---|
| 1 | 11 |
| 2 | 25 |
| 3 | 13 |
| 4 | 20 |
| 5 | -- |
| Comparative Example | |
| 1 | 41 |

Table 2 (continued)

| Example | Wet time (sec.) |
|---|---|
| Comparative Example | |
| 2 | 40 |
| 3 | -- |

Since the CWSTs of the leukocyte eliminating filters according to this invention represented by these Examples were within the range of 90 to 95 dyn/cm as seen from the above results, the operation of priming by making use of physiological saline would not be required, and the time required for the filtration of blood can be shortened, so that the time for emergency blood transfusion or for manufacturing a blood preparation can be shortened.

Additionally, since the leukocyte eliminating filters represented by these Examples were capable of exhibiting excellent performance in eliminating leukocytes and platelets without troublesome treatment of the filters, the manufacture of the filters has been simplified, thus improving the productivity thereof.

Furthermore, since the leukocyte eliminating filters represented by these Examples were substantially free from any eluted material, the filters can be employed safely.

## Claims

1. A leukocyte filter formed of a porous substrate, wherein the substrate is coated with at least one of polymers (I) and (II):

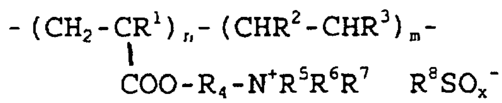

(I)

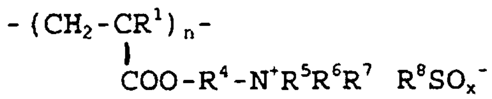

(II)

wherein $R^1$, $R^3$, $R^4$, $R^5$, $R^6$ and $R^7$ may be the same or different and are individually hydrogen atom or an alkyl group having 1 to 4 carbon atoms; $R^2$ is $CONH_2$ or COOH; $R^8$ is an alkyl group having 1 to 12 carbon atoms; and x is 3 or 4.

2. A filter according to claim 1, wherein the critical wet surface tension (CWST) of said filter is in the range 90 to 95 dyn/cm.

3. A filter according to claim 1 or claim 2, wherein said porous substrate is a polyurethane porous body 0.1 to 30µm in average pore diameter, and 75% to 95% in porosity.

4. A method of manufacturing a leukocyte eliminating filter formed of a porous film, which comprises the steps of:

   coating a surface of said porous film with at least one of the polymers formulae (I) and (II) given and defined in claim 1, to form a coating; and
   heat-drying said coating.

5. A method according to claim 4, wherein the critical wet surface tension (CWST) of said filter is in the range 90 to 95 dyn/cm.

6. A method according to claim 4 or claim 5, wherein said porous film is formed of a polyurethane porous body 0.1 to 30µm in average pore diameter, and 75% to 95% in porosity.

**7.** Use of an antistatic agent of the formula I or II given and defined in claim 1 as a coating agent for a leukocyte eliminating filter.

## Patentansprüche

**1.** Leukozytenfilter, gebildet auf einem porösen Substrat, wobei das Substrat mit wenigstens einer der Polymere (I) und (II) beschichtet ist:

$$\begin{array}{l} -(CH_2-CR^1)_n-(CHR^2-CHR^3)_m- \\ \quad | \\ \quad COO-R_4-N^+R^5R^6R^7\ \ R^8SO_x^- \end{array} \qquad (I)$$

$$\begin{array}{l} -(CH_2-CR^1)_n- \\ \quad | \\ \quad COO-R^4-N^+R^5R^6R^7\ \ R^8SO_x^- \end{array} \qquad (II)$$

wobei $R^1$, $R^3$, $R^4$, $R^5$, $R^6$ und $R^7$ die gleichen oder unterschiedlich sein können und einzeln ein Wasserstoffatom oder eine Alkylgruppe mit 1 bis 4 Kohlenstoffatomen sind; $R^2$ ist $CONH_2$ oder COOH; $R^8$ ist eine Alkylgruppe mit 1 bis 12 Kohlenstoffatomen; und x ist 3 oder 4.

**2.** Filter nach Anspruch 1, wobei die kritische feuchte Oberflächenspannung (CWST) des Filters im Bereich von 90 bis 95 dyn/cm ist.

**3.** Filter nach Anspruch 1 oder Anspruch 2, wobei das poröse Substrat ein poröser Polyurethankörper mit einem mittleren Porendurchmesser von 0,1 bis 30 µm und einer Porosität von 75 bis 95% ist.

**4.** Verfahren für die Herstellung eines Leukozyteneliminierenden Filters gebildet auf einem porösen Film, welches die Schritte umfasst von:

Beschichten einer Oberfläche des porösen Films mit wenigstens einem der in Anspruch 1 angegebenen und definierten Polymere der Formeln (I) und (II), um eine Beschichtung zu bilden; und
Wärmetrocknen der Beschichtung.

**5.** Verfahren nach Anspruch 4, wobei die kritische feuchte Oberflächenspannung (CWST) des Filters in dem Bereich von 90 bis 95 dyn/cm ist.

**6.** Verfahren nach Anspruch 4 oder 5, wobei der poröse Film aus einem porösen Polyurethankörper mit einem mittleren Porendurchmesser von 0,1 bis 30 µm und einer Porosität von 75% bis 95% gebildet wird.

**7.** Verwendung eines antistatischen Mittels der in Anspruch 1 angegebenen und definierten Formel I oder II, als ein Beschichtungsmittel für einen Leukozyteneliminierenden Filter.

## Revendications

**1.** Filtre à leucocytes, formé d'un substrat poreux, lequel substrat porte un revêtement d'au moins l'un des polymères de formules (I) et (II) :

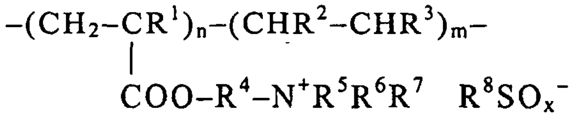

(I)

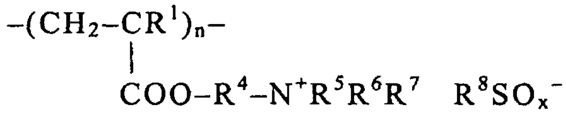

(II)

dans lesquelles $R^1$, $R^3$, $R^4$, $R^5$, $R^6$ et $R^7$, qui peuvent être identiques ou différents, représentent chacun un atome d'hydrogène ou un groupe alkyle comportant 1 à 4 atomes de carbone, $R^2$ représente un groupe -COOH ou $-CONH_2$, $R^8$ représente un groupe alkyle comportant 1 à 12 atomes de carbone, et x vaut 3 ou 4.

**2.** Filtre conforme à la revendication 1, dont la tension critique de mouillage (TCM) vaut de 90 à 95 dyn/cm.

**3.** Filtre conforme à la revendication 1 ou 2, dans lequel ledit substrat poreux est un corps poreux en polyuréthane où le diamètre moyen des pores vaut de 0,1 à 30 µm et dont la porosité vaut de 75 à 95 %.

**4.** Procédé de fabrication d'un filtre retenant les leucocytes, formé d'un film poreux, lequel procédé comporte les étapes suivantes :

- étaler sur la surface dudit film poreux au moins l'un des polymères de formules (I) et (II), indiquées et définies dans la revendication 1, pour en faire un revêtement ;
- et faire sécher ce revêtement en le chauffant.

**5.** Procédé conforme à la revendication 4, dans lequel la tension critique de mouillage (TCM) du filtre vaut de 90 à 95 dyn/cm.

**6.** Procédé conforme à la revendication 4 ou 5, dans lequel ledit film poreux est formé d'un corps poreux en polyuréthane où le diamètre moyen des pores vaut de 0,1 à 30 µm et dont la porosité vaut de 75 à 95 %.

**7.** Emploi d'un agent antistatique de formule (I) ou (II), indiquée et définie dans la revendication 1, comme agent de revêtement pour filtre retenant les leucocytes.